(19) Europäisches Patentamt
European Patent Office
Office européen des brevets

(11) Numéro de publication: **0 247 953 B1**

(12) # FASCICULE DE BREVET EUROPEEN

(45) Date de publication du fascicule du brevet: **13.09.89**

(51) Int. Cl.4: **C07C 149/267**, C07C 148/00, C07C 143/70

(21) Numéro de dépôt: **87420137.9**

(22) Date de dépôt: **20.05.87**

(54) **Procéde de préparation du sulfure de benzyle et de trifluorométhyle.**

(30) Priorité: **28.05.86 FR 8607854**

(43) Date de publication de la demande: **02.12.87 Bulletin 87/49**

(45) Mention de la délivrance du brevet: **13.09.89 Bulletin 89/37**

(84) Etats contractants désignés: **AT BE CH DE ES FR GB GR IT LI LU NL SE**

(56) Documents cités:
**US-A-3 129 262**

**JOURNAL OF ORGANIC CHEMISTRY, Band 46, 1981, Seiten 1938-1940, American Chemical Society, Easton, US; T. NGUYEN et al.: "Reaction of perfluoroalkyl carbanions with thiocyanates. Synthesis of fluorinated sulfides and sulfenyl chlorides"**

(73) Titulaire: **RHONE-POULENC CHIMIE, 25, quai Paul Doumer, F-92408 Courbevoie Cédex(FR)**

(72) Inventeur: **Francese, Catherine, 111 rue de Chevilly, F-94240 L'Hay les Roses(FR)**
Inventeur: **Tordeux, Marc, 1 rue de Seignelay, F-92330 Sceaux(FR)**
Inventeur: **Wakselman, Claude, 5 rue Chanez, F-75016 Paris(FR)**

(74) Mandataire: **Le Pennec, Magali et al, RHONE-POULENC INTERSERVICES Service Brevets Chimie 25, Quai Paul Doumer, F-92408 Courbevoie Cédex(FR)**

EP 0 247 953 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne un nouveau procédé de préparation du sulfure de benzyle et de trifluorométhyle. Elle concerne aussi un procédé de préparation de chlorure de trifluorométhanesulfonyle en deux étapes en passant par l'intermédiaire du sulfure de benzyle et de trifluorométhyle.

Les procédés connus jusqu'à ce jour de préparation du sulfure de benzyle et de trifluorométhyle sont peu nombreux.

Il est connu d'après LEWIS, Mac LAUGHLIN, DOUGLAS (J. Am. Chem. Soc. 1985, 107, 6668-6673) un procédé de préparation du sulfure de benzyle et de trifluorométhyle qui consiste à mettre en contact dans l'ammoniac liquide en présence de rayonnements l'iodure de trifluorométhyle et un benzylmercaptan. Ce procédé praticable au stade du laboratoire n'est en aucune façon envisageable au stade industriel.

Il est aussi connu d'après NGUYEN, RUBINSTEIN, WAKSELMAN (J. Org. Chem. 1981, 46, 1938) un procédé de préparation de sulfures perfluoroalkylés par réaction d'un réactif de Grignard perfluoroalkylé de formule générale $R_FMgX$ dans laquelle $R_F$ représente la partie perfluoroalkyle et X un halogène avec un thiocyanate de benzyle dans l'éther éthylique à une température de -20°C. Cette réaction n'est pas applicable aux dérivés perfluorométhylés car les réactifs de Grignard perfluorométhylés ne sont pas disponibles sur le marché.

La présente invention a permis d'atteindre un procédé industrialisable de préparation de sulfure de benzyle et de trifluorométhyle à partir de matières premières disponibles sur le marché industriel. Elle a permis aussi à partir du sulfur de benzyle et de trifluorométhyle obtenu de le transformer en un produit fortement demandé par le marché qui est le chlorure de trifluorométhane sulfonyle.

La présente invention concerne donc un procédé de préparation de sulfure de benzyle et de trifluorométhyle caractérisé en ce que l'on met en contact dans un solvant aprotique polaire, ou une pyridine éventuellement substituée, un thiocyanate de benzyle, du bromure de trifluorométhyle en présence de zinc.

On entend au sens de la présente invention par sulfure de benzyle et de trifluorométhyle les composés répondant à la formule générale (I)

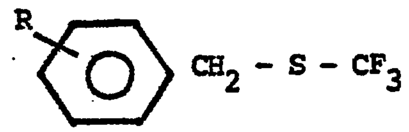

dans laquelle R représente un groupe choisi parmi les radicaux hydrogène, alkyle, alcoxy, alkylthio, aryl, aryloxy, arylthio, halogéno, cyano.

On préfère préparer selon le procédé de l'invention le sulfure de benzyle et de trifluorométhyle non substitué.

Les thiocyanates de benzyle utilisés comme matière première dans le procédé de l'invention répondent à la formule générale (II)

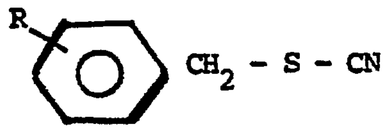

dans laquelle R a la même signification que précédemment.

On préfère utiliser comme matière première le thiocyanate de benzyle non substitué car il est disponible sur le marché et se présente donc d'un point de vue économique sous de bons aspects.

Il est aisément préparé par réaction du thiocyanate de sodium ou de potassium sur, par exemple, le bromure ou le chlorure de benzyle.

Le zinc est avantageusement utilisé sous forme dispersée de façon à assurer le meilleur contact avec le gaz mis en oeuvre dans le procédé de l'invention. La forme et la dimension des particules de métal seront adaptées par l'homme de l'art à la réactivité des produits mis en oeuvre.

Le solvant choisi doit, autant que possible, permettre de solubiliser le thiocyanate de benzyle et le bromure de trifluorométhyle. Répondent à cette condition les pyridines éventuellement substituées et les solvants aprotiques polaires et parmi eux préférentiellement :

- l'acétonitrile
- le diméthylformamide (D.M.F.)
- le diméthylacétamide (D.M.A.)
- l'hexaméthylphosphoramide (H.M.P.A.)
- le diméthylsulfoxyde (D.M.S.O.)
- la N-méthylpyrrolidone (N.M.P.)

La pyridine ou le diméthylformamide sont utilisés encore plus préférentiellement

Selon un mode tout à fait privilégié de mise en oeuvre de l'invention, on utilise un rapport molaire du zinc au thiocyanate de benzyle supérieur ou égal à 1 et un rapport molaire du bromure de trifluorométhyle au thiocyanate de benzyle égal ou supérieur à 1. Si le bromure de trifluorométhyle est mis en excès, celui-ci sera aisément recyclé car il se présente sous forme gazeuse. Il est néanmoins avantageux pour augmenter la solubilité du gaz dans le milieu réactionnel d'opérer sous une pression de bromure de trifluorométhyle comprise entre 1 et 50 bars.

On opère de préférence en l'absence d'oxygène.

La température réactionnelle peut varier dans de larges limites. Cependant une température comprise entre 0 et 100°C est tout à fait favorable pour ce genre de réaction.

La quantité de solvant utilisé n'est pas du tout critique pour la mise en oeuvre de la réaction. Elle sera simplement adaptée par l'homme de l'art à la solubilité du produit de départ.

Les produits issus de la présente invention sont éventuellement séparés par extraction à l'aide de solvants organiques, lavage à l'eau, séchage et distillation.

La préparation de chlorure de trifluorométhane sulfonyle fait également partie de l'objet de la présente invention.

En effet le sulfure de benzyle et de trifluorométhyle obtenu peut être transformé par "chloration oxydante" en chlorure de trifluorométhane sulfonyle. On entend au sens de la présente invention par "chloration oxydante" l'action conjointe du chlore et de l'eau selon le réaction chimique suivante :

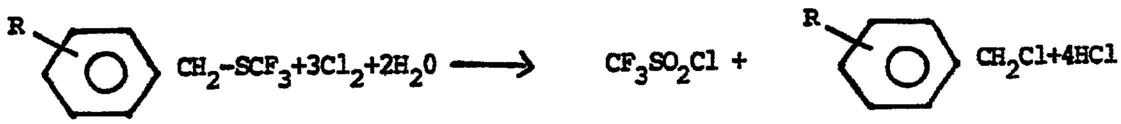

La réaction est mise en oeuvre de préférence à une température comprise entre 0 et 30°C en présence d'une quantité de chlore au moins stoechiométrique. On opère ainsi favorablement en utilisant une pression de chlore comprise entre 1 et 10 bars.

Le chlorure de trifluorométhane sulfonyl est aisément récupéré du milieu réactionnel par simple décantation réalisée à une température comprise entre -15 et 10°C.

Le chlorure de benzyle, sous produit de la réaction, est éventuellement recyclé comme matière première lors de la synthèse du thiocyanate de benzyle.

Les produits objets du procédé de l'invention sont notamment utilisés comme intermédiaires de synthèse dans l'industrie pharmaceutique ou phytosanitaire (J 58 128 343).

L'invention va être plus complètement décrite à l'aide des exemples suivants qui ne devront pas être considérés comme limitatifs de l'invention.

<u>EXEMPLE</u>

<u>1re étape</u>

Dans un ballon on met 25 ml de pyridine, 8 g de benzyl thiocyanate (0,0537 mole) et 4 g de zinc en poudre (0,0615 mole). On purge à l'Azote et on fait passer un courant de bromotrifluorométhane en agitant la solution ; la réaction est exothermique.

On filtre, puis on hydrolyse le filtrat par 20 ml d'acide chlorhydrique à 10 % glacé en agitant environ 30 mn. Après extraction à l'éther, lavage à l'eau, séchage sur sulfate de magnésium et élimination du solvant, le sulfure de benzyle et de trifluorométhyle est distillé sous vide :

Eb = 54-56°C/11 mmHg. On obtient 2,3 g (22 %).
(Eb litt = 76-77°C/30 mmHg).
RMN$^{19}$F (CF $Cl_3$ ext.) : -41,3 ppm
RMN$^{1}$H (TMS int.) 7,3 ppm (m, $C_6H_5$)
4,1 ppm (s, $CH_2$)

<u>2e étape</u>

$$CF_3\ S\ CH_2\ C_6H_5 \xrightarrow[H_2O]{Cl_2} CF_3\ SO_2\ Cl$$

Dans un flacon en verre épais, on met 1,6 g de sulfure de benzyle et de trifluorométhyle (0,0083 mole) et 10 ml d'eau distillée.

Le flacon est placé dans un appareil de Parr. On y fait le vide puis on introduit du chlore gazeux jusqu'à une pression de 4 bars. On agite pendant 2 h, la température étant maintenue entre 0 et 10°C.

Le brut de manipulation est ensuite décanté à froid et on récupère la phase inférieure : le chlorure de trifluorométhane sulfonyle est isolé avec 70 % de rendement.

Eb = 29-32°C/760 mmHg

RMN$^{19}$F ($CFCl_3$ ext.) = -74 ppm

Lorsque la deuxième étape est réalisée en l'absence d'eau et en remplaçant l'eau par 10 ml de tétrachloro 1,1,2,2 éthane on obtient du chlorotrifluorométhylsulfure avec un rendement de 62 % (Eb = 1°C)

## Revendications

1. Procédé de préparation de sulfure de benzyle et de trifluorométhyle caractérisé en ce que l'on met en contact dans un solvant aprotique polaire, ou une pyridine éventuellement substituée, un thiocyanate de benzyle, du bromure de trifluorométhyle en présence de zinc.

2. Procédé selon la revendication 1 caractérisé en ce que le thiocyanate de benzyle répond à la formule générale (II) suivante :

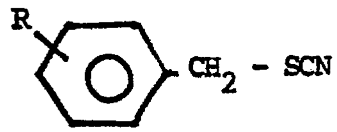

(II)

dans laquelle R représente un groupe choisi parmi les radicaux hydrogène, alkyle, alcoxy, alkylthio, aryl, aryloxy, arylthio, halogéno, cyano.

3. Procédé selon la revendication 2 caractérisé en ce que la formule (II) R représente l'hydrogène.

4. Procédé selon la revendication 1 caractérisé en ce que le solvant aprotique polaire est choisi parmi le diméthylformamide, le diméthylsulfoxyde, l'acétonitrile, l'hexaméthylphosphoramide, le diméthylacétamide, la N-méthylpyrrolidone.

5. Procédé selon la revendication 4 caractérisé en ce que le solvant aprotique polaire choisi est le diméthylformamide.

6. Procédé selon la revendication 1 caractérisé en ce que le solvant choisi est la pyridine.

7. Procédé selon la revendication 1 caractérisé en ce que le rapport molaire du zinc au thiocyanate de benzyle est supérieur ou égal à 1.

8. Procédé selon la revendication 1 caractérisé en ce que le rapport molaire du bromure de trifluorométhyle au thiocyanate de benzyle est supérieur ou égal à 1.

9. Procédé selon la revendication 1 caractérisé en ce que la température réactionnelle est comprise entre 0 et 100°C.

10. Procédé selon la revendication 1 caractérisé en ce que la pression réactionelle est comprise entre la pression atmosphérique et 50 bars.

11. Procédé de préparation du chlorure de trifluorométhane sulfonyle caractérisé en ce que dans une première étape on met en contact dans un solvant aprotique polaire, ou une pyridine éventuellement substituée, un thiocyanate de benzyle, du bromure de trifluorométhyle en présence de zinc,
dans une deuxième étape on met en contact le produit issu de la première étape avec du chlore dans l'eau.

12. Procédé selon la revendication 11 caractérisé en ce que dans la deuxième étape on met en oeuvre une pression de chlore comprise entre 1 et 10 bars.

## Patentansprüche

1. Verfahren zur Herstellung von Benzyltrifluormethylsulfid, dadurch gekennzeichnet, daß man in einem aprotischen polaren Lösungsmittel oder einem gegebenenfalls substituierten Pyridin ein Benzylthiocyanat und Bromtrifluormethan in Gegenwart von Zink miteinander in Berührung bringt.

2. Verfahren nach Anspruch 1, dadurch gekennzeichnet, daß das Benzylthiocyanat der nachstehenden allgemeinen Formel (II) entspricht

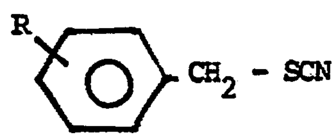

(II)

worin R eine aus den Resten Wasserstoff, Alkyl, Alkoxy, Alkylthio, Aryl, Aryloxy, Arylthio, Halogen, Cyan ausgewählte Gruppe darstellt.

3. Verfahren nach Anspruch 2, dadurch gekennzeichnet, daß in der Formel (II) R Wasserstoff darstellt.

4. Verfahren nach Anspruch 1, dadurch gekennzeichnet, daß das aprotische polare Lösungsmittel unter Dimethylformamid, Dimethylsulfoxid, Acetonitril, Hexamethylphosphoramid, Dimethylacetamid, N-Methylpyrrolidon ausgewählt wird.

5. Verfahren nach Anspruch 4, dadurch gekennzeichnet, daß das gewählte aprotische polare Lösungsmittel Dimethylformamid ist.

6. Verfahren nach Anspruch 1, dadurch gekennzeichnet, daß das gewählte Lösungsmittel Pyridin ist.

7. Verfahren nach Anspruch 1, dadurch gekennzeichnet, daß das molare Verhältnis von Zink zu Benzylthiocyanat oder gleich 1 ist.

8. Verfahren nach Anspruch 1, dadurch gekennzeichnet, daß das molare Verhältnis von Bromtrifluormethan zu Benzylthiocyanat größer oder gleich 1 ist.

9. Verfahren nach Anspruch 1, dadurch gekennzeichnet, daß die Reaktionstemperatur zwischen 0 und 100°C liegt.

10. Verfahren nach Anspruch 1, dadurch gekennzeichnet, daß der Reaktionsdruck zwischen dem atmosphärischen Druck und 50 bar liegt.

11. Verfahren zur Herstellung von Trifluormethansulfonylchlorid, dadurch gekennzeichnet, daß man in einem ersten Schritt in einem aprotischen polaren Lösungsmittel oder einem gegebenenfalls substituierten Pyridin ein Benzylthiocynat mit Bromtrifluormethan in Gegenwart von Zink in Berührung bringt und dann in einem zweiten Schritt das Produkt des ersten Schritts mit Chlor in Wasser in Berührung bringt.

12. Verfahren nach Anspruch 11, dadurch gekennzeichnet, daß im zweiten Schritt bei einem Chlordruck zwischen 1 und 10 bar gearbeitet wird.

## Claims

1. A process for the preparation of benzyl trifluoromethyl sulfide, characterised in that a benzyl thiocyanate and trifluoromethyl bromide are brought into contact with zinc in a polar aprotic solvent or an optionally substituted pyridine.

2. The process according to claim 1, characterised in that the benzyl thiocyanate is of the following general formula (II):

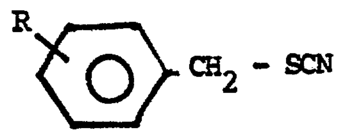 (II)

in which R represents a group chosen from amongst hydrogen, alkyl, alcoxy, alkylthio, aryl, aryloxy, arylthio, halo and cyano radicals.

3. The process according to claim 2, characterised in that in formula (II) R represents hydrogen.

4. The process according to claim 1, characterised in that the polar aprotic solvent is chosen from amongst dimethylformamide, dimethyl sulphoxide, acetonitrile, hexamethylphosphoramide, dimethylacetamide and N-methylpyrrolidone.

5. The process according to claim 4, characterised in that the polar aprotic solvent chosen is dimethylformamide.

6. The process according to claim 1, characterised in that the solvent chosen is pyridine.

7. The process according to claim 1, characterised in that the molar ratio of zinc to benzyl thiocyanate is greater than or equal to 1.

8. The process according to claim 1, characterised in that the molar ratio of trifluoromethyl bromide to benzyl thiocyanate is greater than or equal to 1.

9. The process according to claim 1, characterised in that the reaction temperature is between 0 and 100°C.

10. The process according to claim 1, characterised in that the reaction pressure is between the atmospheric pressure and 50 bar.

11. A process for the preparation of trifluoromethanesulphonyl chloride characterised in that, in a first stage, benzyl thiocyanate and trifluoromethyl bromide are brought into contact with zinc in a polar aprotic solvent or an optionally substituted pyridine, and in a second stage, the product resulting from the first stage is brought into contact with chlorine in water.

12. The process according to claim 11, characterised in that in the second stage, a chlorine pressure of between 1 and 10 bar is employed.